# EUROPEAN PATENT SPECIFICATION

(11) **EP 1 490 091 B1**
(45) Date of publication and mention of the grant of the patent: **29.08.2007**
(21) Application number: 03706050.6
(22) Date of filing: 31.01.2003
(51) Int. Cl.: A61K 38/09

(54) **ENHANCEMENT OF ENDOGENOUS GONADOTROPIN PRODUCTION**
VERBESSERUNG DER HERSTELLUNG VON ENDOGENEN GONADOTROPIN
AMELIORATION DE LA PRODUCTION DE LA GONADOTROPINE ENDOGENE

(30) Priority: 28.03.2002 US 63197
(43) Date of publication of application: 29.12.2004
(73) Proprietor: TAP Pharmaceutical Products, Inc., Lake Forest, Illinois 60045-6050 (US)
(72) Inventor: TANEJA, Rajneesh, Libertyville, IL 60048 (US)
(74) Representative: Modiano, Micaela Nadia
(86) International application number: PCT/US2003/003131
(87) International publication number: WO 2003/082319

(56) References cited:
- EP-A- 0 788 799
- EP-A- 0 947 200
- WO-A-00/27193
- WO-A-90/14839
- WO-A-99/55357
- US-A- 5 538 948
- US-A- 5 942 242
- US-B1- 6 265 393
- ZITZMANN M ET AL: "HORMONE SUBSTITUTION IN MALE HYPOGONADISM" CHINESE CHEMICAL LETTERS, XX, XX, vol. 161, no. 8, 30 March 2000 (2000-03-30), pages 73-88, XP001057049 ISSN: 1001-8417

## Description

### Technical Field

The present invention relates to a method of enhancing endogenous sex hormone production utilizing gonadotropin releasing hormone agonists.

### Background of the Invention

Native gonadotropin releasing hormone (GnRH), also known as LH-RH, is a hormone that is secreted by the hypothalamus in a pulsating fashion. Release of GnRH results in a cascade of hormonal events leading to the production of testosterone. Specifically, release of GnRH stimulates the pituitary gland to produce leutininizing hormone (LH) and follicle stimulating hormone (FSH), all of which are considered "gonadotropins". LH and FSH are important for maintaining the normal male and female reproductive functions and act on Leydig cells in the testis to produce testosterone, a so-called "androgen". LH, FSH, and testosterone are sometimes referred to as "sex hormones".

Testosterone produced by the Leydig cells is further converted to dihydrotestosterone (DHT) by 5α-reductase enzyme. DHT is acted upon by an enzyme called aromatase that converts it into estradiol. About 98% of the testosterone present in the blood is available in a bound form primarily to sex hormone binding protein (SHBG) and, to a lesser extent, to albumin and cortisol binding globulin. Testosterone and estradiol cause feedback inhibition at the pituitary and hypothalamus level, and therefore when their concentration is at a sufficient level, they inhibit release of GnRH from the hypothalamus.

Administration of native GnRH has been found to increase sex hormone production. Belchetz PE, et. al.; Responses to Continuous and Intermittent Delivery of Hypothalamic Gonadotropin-Releasing Hormone, Science 1978; 202:631-633, demonstrated that intermittent, but not continuous, administration of GnRH resulted in increases in plasma LH and FSH levels. In another study (Rommler, et. al.; LH-Rh Double Stimulation Technique in the Differential Diagnosis of Amenorrhea, Acta Endocr. (Kbh) Suppl. 1973; 177-292) double stimulation tests with 25 mcg LH-RH each on 3 consecutive days were performed on 12 normally menstruating females. Increases in serum LH levels were found after each of the two stimulations. The second LH increase was as high as the first increase when the interval between the injections was 6 hours or more. The second surge was distinctively higher than the first one when the interval between injections was 1 and 4 hours. Schneider, et al.; Evidence for Partial Refractoriness of the Human Pituitary. Acta. Endocr. (Kbh) Suppl. 1973; 173-86, reported similar findings. In light of these and similar findings, native GnRH has been therapeutically employed to increase gonadotropin production which, in turn, stimulates testosterone production. Unfortunately, native GnRH generally is administered in an outpatient setting and is not available for convenient or self administration.

GnRH agonists (variously referred to as "LH RH agonists") are compounds that mimic native GnRH in structure, but not necessarily in function. Initially, GnRH agonists were considered as potential therapy for male and female infertility. It was postulated that due to the structural similarity between native GnRH and GnRH agonists, that the agonists would increase sex hormone concentrations similarly to native GnRH. Unfortunately, animal and human studies showed that chronic administration of GnRH agonists resulted in suppression of sex hormone concentrations. Hence, GnRH agonists were found to have the opposite effect of native GnRH. Accordingly, GnRH agonists traditionally have been employed for reducing the levels of sex hormones such as estrogen, progesterone and testosterone. In fact, GnRH agonists have been used therapeutically to, for example, starve androgen dependent tumors because of their potent ability to reduce testosterone concentrations to near castration levels.

A number of different mechanisms may be involved in the reduction in gonadotropin and androgen levels associated with GnRH agonists. It has been postulated that continuous therapy with GnRH agonists results in a decrease in the number of pituitary GnRH and/or testicular LH receptors that results in pituitary and/or testicular desensitization, respectively. It also has been suggested that LH molecules secreted as a result of GnRH agonist stimulation have an altered biological activity. However, no theory has been universally accepted.

There is therefore a need for a therapy capable of increasing gonadotropin and/or androgen levels for extended periods that can be delivered relatively easily.

### Summary of the Invention

The present invention provides methods for enhancing or increasing the production of, or otherwise increasing the levels of, endogenous gonadotropins and/or androgens. The method comprises administering a therapeutically effective amount of a GnRH agonist to a patient in need of such therapy to thereby increase the levels of, for example, follicle stimulation hormone (FSH), leutinizing hormone (LH), and testosterone. Any suitable route of administration can be employed.

### Detailed Description of the Invention

As previously mentioned, GnRH agonists historically have been used therapeutically to decrease so-called sex hormone concentrations such as LH, FSH, and testosterone. It has now been discovered that through appropriate dosing, GnRH agonists may be employed to increase, or enhance, sex hormone levels. Hence, therapies for diseases associated with deficiencies in gonadotropin, androgen, or a combination of both, are provided.

Methods for enhancing gonadotropin levels, androgen levels, or both comprise administering a therapeutically effective amount of a GnRH agonist to a patient in need of such therapy. Diseases that are appropriate for such therapy are hypogonadism in aging males, cryptochidism, male infertility, amenorrhea and female infertility, as well as decreased libido, particularly in male patients. Additionally, the methods herein can be employed to improve existing therapies. For example, sexual dysfunction, such as erectile dysfunction, is currently treated with phosphodiesterase inhibitors and dopamine agonists that alleviate physiological aspects of the disease. However, sexual dysfunction has been found to have a psychological component as well. Moreover, increased testosterone levels have an impact on the psychological component of sexual dysfunction. Hence, the methods provided herein would benefit the existing therapy for sexual dysfunction.

A "therapeutically effective amount", as used herein, means an amount of a GnRH agonist given to a patient at a frequency that increases the levels of LH, FSH, or testosterone in a patient without decreasing the normal levels of those hormones in the patient receiving such therapy. The specific therapeutically effective amount for any particular patient will depend upon a variety of factors including the disorder being treated; the severity of the disorder; the activity of the specific compound employed; the specific composition employed; the age, body weight, general health, sex and diet of the patient; the time of administration; route of administration; the rate of excretion of the specific compound employed; the duration of the treatment; the drugs used in combination or coincidental with the specific compound employed; and other factors known to those of ordinary skill in the medical arts. For example, it is well within the skill of the art to start doses of the compound at levels lower than required to achieve the desired therapeutic effect and to gradually increase the dosage until the desired effect is achieved. The levels of the hormones whose concentrations are sought to be increased also are easily measured using commercially available diagnostic assays which can be employed to establish a baseline hormone level, an increase in hormone level above baseline, and a decrease in hormone level below baseline. These parameters can be employed to appropriately dose a particular patient such that a patient receives the desired effect.

A pulsed dosing regimen of a GnRH agonist has been found to increase sex hormone levels without experiencing a decrease in sex hormone levels seen with typical GnRH dosing regimens. In particular, a "pulsed dosing" is one where there is a period of GnRH administration and a period between the next administration period sufficient in time to allow a patient to clear the GnRH agonist from their body. In a typical administration period, between one and four doses of a GnRH agonist are provided to the patient. Most typically, one to two doses of the GnRH agonist are provided in an administration period. The period between individual doses in an administration period can be between 1 hour and eight hours, more typically between one hour and four hours. Typically, GnRH remains in humans for approximately 48 hours. Accordingly, the period between administration periods is preferably between 36 hours and 60 hours, depending upon the individual. Additionally, the course of therapy may be for so long as a patient requires an increase in sex hormone levels. Typically, such therapy will be for more than one week, preferably two or more weeks and most preferably one month. Dosing levels for the GnRH agonist may be between 5 mcg and 1500 mcg by a parenteral mode of administration, between 500 mcg and 15 mg by a sublingual mode of administration and 125 mcg and 3.75 mg by an inhalation route of administration. Such doses preferably are provided twice a day every other day with the doses on a particular day being between one hour and eight hours apart.

GnRH agonists are well known and include, but are not limited to, leuprolide acetate, buserelin, naferelin, deslorein, histerelin, goserelin, cetrorelix. The GnRH agonists can be in a variety of well known formulations and administered using any of a variety of well known methods of administration. Thus, the GnRH agonists may be, for example, part of a liquid, gel, solid, powder, or suspension type formulation which can be administered through injection, inhalation, transdermally, orally, or the like. Preferably, the GnRH agonists are administered in a "non-invasive" manner (i.e. without puncturing the patient such as when drugs are administered through injection). Hence, non-injectable formulations are preferred and inhaled formulations are particularly preferred.

The GnRH agonists may be included in a pharmaceutical compositions comprising nontoxic physiologically tolerable or acceptable carriers, adjuvants or vehicles that are collectively referred to herein as diluents. Acceptable adjuvants include preserving, wetting emulsifying, and dispensing agents; acceptable carriers include solvents, bulk formers, and fillers; and acceptable vehicles include waters, elixirs, syrups, hydroalcohols, mucilages, oils, glycol ethers and derivatives thereof. Prevention of the action of microorganisms may also be desirable in a formulation of a GnRH agonist and can be ensured by various antibacterial and antifungal agents, for example, parabens, chlorobutanol, phenol, sorbic acid, and the like. It may also be desirable to include isotonic agents, for example sugars and sodium chloride, among others.

In cases where the GnRH agonist is included in a suspension the formulation may contain suspending agents, as for example, ethoxylated isostearyl alcohols, polyoxyethylene sorbitol and sorbitan esters, microcrystalline cellulose, aluminum metahydroxide, bentonite, agar-agar and tragacanth, or mixtures of these substances, among others.

Useful intranasal formulations of a GnRH agonist may contain a stabilizers and a surfactants. Among the pharmaceutically acceptable surfactants are polyoxyethylene castor oil derivatives, such as polyoxyethylene-glycerol-triricinoleate, also known as polyoxyl 35 caster oil (CREMOPHOR EL), or poloxyl 40 hydrogenated castor oil (CREMOPHOR RH40) both available from BASF Corp.; mono-fatty acid esters of polyoxyethylene (20) sorbitan, such as polyoxyethylene (20) sorbitan monolaurate (TWEEN 80), polyoxyethylene monostearate (TWEEN 60), polyoxyethylene (20) sorbitan monopalmitate (TWEEN 40), or polyoxyethylene 20 sorbitan monolaurate (TWEEN 20) (all available from ICI Surfactants of Wilmington, DE); polyglyceryl esters, such as polyglyceryl oleate; and polyoxyethylated kernel oil (LABRAFIL, available from Gattefosse Corp.). Preferably, the surfactant will be between about 0.01% and 10% by weight of the pharmaceutical composition.

Among the pharmaceutically useful stabilizers are antioxidants such as sodium sulfite, sodium metabisulfite, sodium thiosulfate, sodium formaldehyde sulfoxylate, sulfur dioxide, ascorbic acid, isoascorbic acid, thioglycerol, thioglycolic acid, cysteine hydrochloride, acetyl cysteine, ascorbyl palmitate, hydroquinone, propyl gallate, nordihydroguaiaretic acid, butylated hydroxytoluene, butylated hydroxyanisole, alpha-tocopherol and lecithin. Preferably, the stabilizer will be between about 0.01 % and 5% by weight of the pharmaceutical composition.

Suspensions may also include chelating agents such as ethylene diamine tetraacetic acid, its derivatives and salts thereof, dihydroxyethyl glycine, citric acid and tartaric acid among others. Additionally, proper fluidity of a suspension can be maintained, for example, by the use of coating materials such as lecithin, by the maintenance of the required particle size in the case of dispersions and by the use of surfactants, such as those previously mentioned.

Solid dosage forms for oral administration include capsules, tablets, pills, powders and granules. In such solid dosage forms, the active compound may be mixed with at least one inert, pharmaceutically acceptable excipient or carrier, such as sodium citrate or dicalcium phosphate and/or (a) fillers or extenders such as starches, lactose, sucrose, glucose, mannitol and silicic acid; (b) binders such as carboxymethylcellulose, alginates, gelatin, polyvinylpyrrolidone, sucrose and acacia; (c) humectants such as glycerol; (d) disintegrating agents such as agar-agar, calcium carbonate, potato or tapioca starch, alginic acid, certain silicates and sodium carbonate; (e) solution retarding agents such as paraffin; (f) absorption accelerators such as quaternary ammonium compounds; (g) wetting agents such as cetyl alcohol and glycerol monostearate;(h) absorbents such as kaolin and bentonite clay; and (i) lubricants such as talc, calcium stearate, magnesium stearate, solid polyethylene glycols, sodium lauryl sulfate and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like.

The solid dosage forms of tablets, capsules, pills and granules can be prepared with coatings and shells such as enteric coating and other coatings well-known in the pharmaceutical formulating art. They may optionally contain opacifying agents and may also be of a composition such that they release the active ingredient(s) only, or preferentially, in a certain part of the intestinal tract, optionally, in a delayed manner. Examples of embedding compositions which can be used include polymeric substances and waxes.

Liquid dosage forms for oral administration include pharmaceutically acceptable emulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils (in particular, cottonseed, groundnut, corn, germ, olive, castor and sesame oils), glycerol, tetrahydrofurfuryl alcohol, polyethylene glycols and fatty acid esters of sorbitan and mixtures thereof.

### Examples

### Example 1

The objectives of this study were to assess the pharmacokinetics, safety profile and hormonal response of fixed (5 mg q.d, 5 mg b.i.d. and 10 mg q.d.) oral leuprolide acetate administered for 28 days to healthy male volunteers. This was a phase 1, single center, fixed-dose open label study where three doses of oral leuprolide acetate were administered to 60 healthy male volunteers for 28 days. Twenty subjects were assigned to each dosing group. Dosing groups were dosed sequentially, beginning with 5 mg q.d. group (Group A). Dosing for the 5 mg b.i.d. group (Group B) commenced 2 weeks after the initiation of Group A and the dosing for the 10 mg q.d. group (Group C) began 2 weeks after initiation of the 5 mg b.i.d. group. The formulation used for dosing the patients consisted of water, ethanol, oleic acid, leuprolide acetate 5mg/ml and tween 80. The final formulation to be dosed was prepared at the investigational site by a pharmacist. For all measurements, the final value obtained before the start of the study drug administration was used as the baseline value for that variable. When applicable, only pre-dose (prior to morning dose for the leuprolide acetate 5 mg b.i.d. group) hormone values were used. The observed plasma testosterone levels during the first 10 days of the study are shown in Table 1.

**Table 1**

| Testosterone levels ng/dL | | | | | | | | | | |
|---|---|---|---|---|---|---|---|---|---|---|
| Leuprolide acetate Dose | Baseline | Day 2 | Day 3 | Day 4 | Day 5 | Day 6 | Day 7 | Day8 | Day 9 | Day 10 |
| Group A | 453.25 | 452.45 | 406.25 | 384.70 | 430.55 | 434.55 | 413.75 | 405.85 | 358.8 | 369.80 |
| Group B | 485.00 | 563.5 | 541.35 | 527.40 | 502.85 | 493.25 | 484.75 | 449.10 | 427.65 | 428.95 |
| Group C | 458.85 | 495.85 | 501.00 | 492.20 | 463.90 | 412.65 | 435.50 | 409.00 | 428.05 | 405.95 |

The testosterone levels for Group B were higher than the baseline until Day 7 of the study. Testosterone levels for Group C were higher than the baseline until Day 6 of the study. When leuprolide acetate administered at a dose of 5 mg q.d. (Group A) failed to raise the levels of testosterone above the baseline, 5 mg b.i.d. (Group B) administration resulted in more significant elevation of plasma testosterone in comparison with 10 mg q.d. dose (Group C). In Groups B and C, the testosterone levels declined below baseline on Day 7 and Day 6 onwards. This was probably due to the exhausted pituitary responsiveness subsequent to multiple stimuli.

### Example 2

Two examples of leuprolide acetate formulation for sublingual administration are provided here:

**Table 2**

| | | |
|---|---|---|
| Alcohol, Dehydrated, USP, 200 Proof | 80% v/v | 57% |
| Hydroxypropyl Cellulose, NF | 2.50% w/v | 2.50% w/v |
| Acid Benzoic, USP | 10% w/v | 10% w/v |
| Leuprolide acetate | 45 mg per ml | 45 mg per ml |
| Oil, Peppermint, NF | | 2% |
| Nitrogen, NF | Q.S. | Q.S. |
| Water, Purified, USP Distilled | Q.S. | Q.S. |

| | | |
|---|---|---|
| Add appropriate amount of Nitrogen saturated Purified Water to a clean, dry tared and suitable size vessel. Add the Alcohol to the Purified Water with mixing. Add the Hydroxypropyl Cellulose to the Water/Alcohol with mixing until dissolved. Add the Benzoic Acid with mixing until dissolved. Add Peppermint Oil if it is included in the formulation and mix well. Carefully add the leuprolide acetate while mixing slowly until dissolved. Add Nitrogen saturated Purified Water to make up the final volume. | | |

### Example 3

A study was conducted to determine the bioavailability of leuprolide acetate when it was administered by the sublingual route. The study was conducted in fifteen healthy postmenopausal or surgically sterilized female volunteers. This study was conducted according to a single-dose, fasting, four-period, open-label, randomized design. Each patient received a single sublingual dose of leuprolide acetate: 1.1.25 mg, 2.25 mg. 4.5 mg or a 1 mg subcutaneous dose. Formulation A described in Example 2 was utilized for all the leuprolide acetate sublingual doses. Lupron □ Injection (TAP Pharmaceuticals Inc., Lake Forest, IL) containing 1 mg leuprolide acetate, sodium chloride for tonicity adjustment and 1.8 mg benzyl alcohol as preservative was utilized for the subcutaneous dose in all subjects. Five-ml plasma samples were collected by venipuncture prior to the study and at suitable time intervals after dosing. Plasma leuprolide acetate concentrations data was tabulated and the descriptive statistics were computed. Approximately 1% of the administration sublingual dose was absorbed compared to subcutaneous reference after the adjustment for the difference in doses.

### Example 4

A study was conducted to determine the extent of absorption after inhalation administration of leuprolide acetate in three aerosol formulations, a 10-mg/ml solution, a 10-mg/ml suspension and a 20-mg/ml suspension. The extent of absorption obtained with each aerosol formulation was compared to the absorption obtained after intravenous administration of a 1 mg dose. The study was an open-label, single-dose, randomized, four-period crossover study involving 24 normal adult male volunteers.. Subjects were randomly assigned into four groups, each of which had a distinct treatment of regimen sequence. By the end of the study, each subject received each of the four different regimens of leuprolide acetate.

**Table 3**

| Group | Period 1 | Period 2 | Period 3 | Period 4 |
|---|---|---|---|---|
| 1 | A | B | C | D |
| 2 | B | D | A | C |
| 3 | C | A | D | B |
| 4 | D | C | B | A |

| | | | | |
|---|---|---|---|---|
| A = 1 mg leuprolide acetate inhalation (solution) aerosol B = 1 mg leuprolide acetate inhalation (suspension) aerosol C = 2 mg leuprolide acetate inhalation (suspension) aerosol D = 1 mg leuprolide acetate iv injection | | | | |

For each dosing period, blood samples were drawn just prior to dosing and at specified intervals through 24-hour post-dosing. Plasma was separated from these samples, labeled in tubes and frozen. At the end of the study, plasma leuprolide acetate levels were determined by radioimmunoassay. The absolute bioavailabilities for the inhalation aerosol dosage forms were approximately: 4%, 18%, and 14% for the 1 mg solution, 1 mg suspension and 2 mg suspension, respectively.

## Claims

1. Use of a GnRH agonist for manufacturing a medicament for treating hypogonadism, cryptochidism, male infertility, female infertility, amenorrhea and male erectile dysfunction by administration of a therapeutically effective amount to a patient in need of such treatment, which amount enhances endogenous gonadotropin and androgen.

2. The use of claim 1 wherein the GnRH agonist is administered non-invasively.

3. The use of claim 2 wherein the GnRH agonist is administered with an oral, nasal, or inhaled dosage form.

4. The use of any of claims 1, 2 or 3 wherein the GnRH agonist is leuprolide acetate, buserelin, naferelin, deslorelin, histerelin, goserelin and cetrorelix.

5. The use of claim 1 wherein the administration is for treating male erectile dysfunction and it further comprises administration of a phosphodiesterase inhibitor or a dopamine agonist.

## Patentansprüche

1. Verwendung eines GnRH Agonisten für die Herstellung eines Medikaments zur Behandlung von Hypogonadismus, Cryptorchidismus, männlicher Infertilität, weiblicher Infertilität, Ammenorrhoe und männlicher erektiler Dysfunktion durch Verabreichen einer therapeutisch wirksamen Menge an einen Patienten, der eine solche Behandlung benötigt, wobei die Menge die endogenen Gonadotropine und Androgene erhöht.

2. Die Verwendung von Anspruch 1, worin der GnRH Agonist nicht-invasiv verabreicht wird.

3. Die Verwendung von Anspruch 2, worin der GnRH Agonist mit einer oralen, nasalen oder inhalierten Dosierform verabreicht wird.

4. Die Verwendung von irgendeinem der Ansprüche 1, 2 oder 3, worin der GnRH Agonist Leuprolidacetat, Buserelin, Naferelin, Deslorelin, Histerelin, Goserelin und Cetrorelix ist.

5. Die Verwendung von Anspruch 1, worin die Verabreichung zur Behandlung von männlicher erektiler Dysfunktion dient, und sie weiter die Verabreichung eines Phosphodiesterase-Inhibitors oder eines Dopaminagonisten umfaßt.

## Revendications

1. Utilisation d'un agoniste de GnRH pour la préparation d'un médicament pour le traitement de l'hypogonadisme, du cryptorchidisme, de l'infertilité masculine, de l'infertilité féminine, de l'aménorrhée et des dysfonctionnements érectiles masculins, par administration d'une quantité thérapeutiquement efficace à un patient nécessitant un tel traitement, laquelle quantité augmente la gonadotrophine et l'androgénie endogènes.

2. Utilisation selon la revendication 1, dans laquelle l'agoniste de GnRH est administré de manière non invasive.

3. Utilisation selon la revendication 2, dans laquelle l'agoniste de GnRH est administré sous une forme posologique orale, nasale ou inhalée.

4. Utilisation selon l'une quelconque des revendications 1, 2 ou 3, dans laquelle l'agoniste de GnRH est l'acétate de leuprolide, la buséréline, la naféréline, la desloréline, l'histéréline, la goséréline et le cetrorelix.

5. Utilisation selon la revendication 1, dans laquelle l'administration est destinée au traitement d'un dysfonctionnement érectile masculin et comprend en outre, l'administration d'un inhibiteur de phosphodiestérase ou d'un agoniste de la dopamine.
